# EUROPÄISCHE PATENTSCHRIFT

(11) **EP 1 082 063 B1**
(45) Veröffentlichungstag und Bekanntmachung des Hinweises auf die Patenterteilung: **01.09.2004**
(21) Anmeldenummer: 99917728.0
(22) Anmeldetag: 11.05.1999
(51) Int. Cl.: A61B 17/68, A61B 17/04, A61F 2/02

(54) **CHIRURGISCHE BLINDNIETE MIT SCHLIESSELEMENT**
SURGICAL BLIND RIVETS WITH CLOSING ELEMENTS
RIVET AVEUGLE CHIRURGICAL DOTE D'UN ELEMENT DE FERMETURE

(30) Priorität: 04.06.1998 WO PCT/CH98/00242
(43) Veröffentlichungstag der Anmeldung: 14.03.2001
(73) Patentinhaber: SYNTHES AG Chur, 7002 Chur (CH)
(72) Erfinder: FRIGG, Robert, CH-2544 Bettlach (CH)
(74) Vertreter: Lusuardi, Werther Giovanni, Dr.
(86) Internationale Anmeldenummer: PCT/CH1999/000194
(87) Internationale Veröffentlichungsnummer: WO 1999/062418

(56) Entgegenhaltungen:
- EP-A- 0 328 314
- EP-A- 0 537 967
- EP-A- 0 747 023
- DE-A- 2 701 279
- DE-A- 3 217 065
- FR-A- 2 141 020
- GB-A- 887 799
- GB-A- 2 054 082
- US-A- 4 736 560

## Beschreibung

Die Erfindung bezieht sich auf eine Vorrichtung zur Fixation von chirurgischen Implantaten, chirurgischen Fäden oder Geweben im oder am Knochen gemäss dem Oberbegriff des Patentanspruchs 1.

Aus dem nicht medizinischen Bereich sind bereits Blindnieten bekannt, deren Schliessköpfe aus separaten, relativ weit aufgespreizten Zungen bestehen. Solche Blindnieten sind beispielsweise aus der US 4,696,610 WRIGHT und der US 4,580,936 FRANCIS bekannt. Diese bekannten Blindnieten weisen längsgerichtete Schlitze oder Schnitte auf, welche die Ausbildung der aus separaten Zungen bestehenden Schliessköpfe beim Schliessen der Blindniete ermöglichen. Die so gebildeten Schliessköpfe weisen eine grosse Auflagefläche auf. Nachteilig bei diesen bekannten Blindnieten ist, dass die Schliessköpfe durch Knicken der längsgerichteten Zungen gebildet werden und somit die Enden der Zungen nicht als scharfe Zacken in das schliesskopfseitig zu verbindende Material einpressbar sind, wodurch eine Rotation der Blindniete in den Bohrungen der zu verbindenden Materialien verhindert werden könnte.

Aus der GB 2 054 082 TUCKER FASTENERS ist eine weitere nicht-medizinische Blindniete bekannt, welche allerdings eine kreisrunde Durchgangsöffnung aufweist, so dass keine Rotationssicherung besteht.

Aus der EP-A-0537967 und der GB-887799 sind schliesslich weitere Nieten für den Industriegebrauch bekannt , insbesondere zur Verwendung in Landfahrzeugen und im Bauwesen, sie sind jedoch für den medizinischen Gebrauch, insbesondere in der Chirurgie ungeeignet.

Aus der EP-A-0747023 ist eine gattungsgemässe Vorrichtung bekannt, welche zur Fixation von chirurgischen Implantaten, chirurgischen Fäden im oder am Knochen dient. Die Konstruktion ist aber kompliziert und speziell nur zur Weichteil-Fixierung vorgesehen.

Hier will die Erfindung Abhilfe schaffen. Der Erfindung liegt die Aufgabe zugrunde, ein Fixationssystem zu schaffen, das durch eine kleine Bohrung eingeführt werden kann und nach der Fixation dank der aufgespreizten Verankerungszungen eine breite Abstützung auf der Knocheninnenfläche hat, wobei sich die Verankerungszungen in die Knocheninnenfläche eingraben können und dadurch eine Rotation der Blindniete verhindern.

Im medizinischen Bereich kann das erfindungsgemässe Fixationselement fast überall in der Knochenfrakturbehandlung eingesetzt werden wo gegenwärtig Knochenschrauben Verwendung finden, speziell:
- Beim porotischen Knochen, der zum Teil nur eine sehr dünne Kortikalis hat, kann das erfindungsgemässe Fixationselement an Stelle von Schrauben zur Plattenfixation verwendet werden;
- in der Spongiosa kann das erfindungsgemässe Fixationselement als Anker für Platten, Nähfäden oder zur Refixation von Sehnen und Bändern verwendet werden. Die sich beim Spreizen des Schliesskopfes der Blindniete bildenden Krallen verankern sich hervorragend in der Bälkchenstruktur der Spongiosa; und
- In Gelenksköpfen, wie Femurkopf oder Humeruskopf, kann das erfindungsgemässe Fixationselement als Verankerung einer Seitenplatte oder eines intramedullären Trägers verwendet werden. Gegenwärtig werden zur Verankerung der Längsträger grosse Schrauben verwendet. Diese Schrauben bieten jedoch keine optimale Verankerung im porotischen Knochen der Gelenksköpfe.

Ähnliche Systeme sind Schrauben, Marknägel oder Hüftschrauben, die ausklinkbare Krallen haben, beispielsweise der Seidel-Marknagel für den Humerus. Dieser Marknagel hat Schlitze an der Nagelspitze, die durch einen Zentralstift mit Kugelkopf gespreizt werden. Dadurch werden Zungen flügelartig nach aussen gebogen. Allerdings werden diese Zungen nur leicht vom Nagel abgewinkelt.

Nachteilig bei diesen bekannten Fixationsmittel ist, dass die Verankerung von Implantaten im porotischen Knochen mit diesen bekannten Fixationsmitteln bis heute nicht gelöst ist. Oft kann ein Implantat nur durch Einspritzen von Knochenzement in den Knochen fixiert werden. Das ist sehr problematisch, da der Knochen beim Aushärten des Zementes durch die entstehende Hitze geschädigt wird. Zudem kann der Zement nicht mehr entfernt werden, was bei einer eventuell auftretenden Infektion notwendig ist.

Ein weiterer Nachteil dieser bekannten Fixationsmittel liegt darin, dass die Haltekraft beispielsweise einer Schraube durch ihren Durchmesser gegeben ist. Bei axialer Überlast wird ein Knochenzylinder, gleich dem Durchmesser der Schraube herausgerissen. Die Haltekraft von Knochenschrauben ist beim gesunden Knochen ausreichend. Bei Osteoporose, in Gelenksbereichen oder beim dünnen, schalenartigen Knochen sind Schraubenfixationen oft unzureichend. Grössere Schrauben können aus Platzgründen nicht verwendet werden, oder zerstören den noch vorhandenen Knochen zusätzlich.

Hier will die Erfindung Abhilfe schaffen. Der Erfindung liegt andererseits die Aufgabe zugrunde, ein Fixationssystem zu schaffen, das durch eine kleine Bohrung eingeführt werden kann und nach der Fixation dank der aufgespreizten Verankerungszungen eine breite Abstützung auf der Knocheninnenfläche hat.

Die Erfindung löst die gestellte Aufgabe mit einer Vorrichtung zur Fixation von chirurgischen Implantaten, chirurgischen Fäden oder Geweben im oder am Knochen, welche die Merkmale des Anspruchs 1 aufweist.

Die Durchgangsöffnung in der Blindniete am schliessenden Teil weist einen polygonalen Querschnitt auf, der vorzugsweise quadratisch ist, aber auch drei-, fünf-, sechs- oder mehreckig sein kann.

Eine bevorzugte Ausführungsform der erfindungsgemässen Vorrichtung zur Fixation von chirurgischen Implantaten, chirurgischen Fäden oder Geweben im oder am Knochen besteht aus einer Blindniete mit einer Längsachse und einer koaxialen Durchgangsöffnung sowie aus einem in die Durchgangsöffnung einführbaren Schliesselement. Das Schliesselement weist mindestens auf einem an sein hinteres Ende anschliessenden Teil seiner Länge einen polygonalen Querschnitt auf, wobei auf diesem Teil seiner Länge der Querschnitt entlang der Längsachse gegen das hintere Ende des Schliesselementes weiter wird. Das Schliesselement wird so in die Blindniete eingebracht, dass der kleinere Querschnitt des konischen Teils des Schliesselementes zuerst so weit in die Blindniete eingeführt wird bis dieses wegen des weiter werdenden Querschnittes in der Durchgangsöffnung der Blindniete ansteht. Beim Schliessen der Blindniete durch weiteres Einpressen des Schliesselementes in die Durchgangsöffnung der Blindniete wird diese auf dem zu schliessenden Teil durch den weiter werdenden polygonförmigen Teil des Schliesselementes in separate Verankerungszungen getrennt, welche in der Folge durch den weiter werdenden Teil des Schliesselementes halbkreisförmig aufgespreizt werden und einen auf einer grossen Fläche verankerten, aus einer Anzahl Verankerungszungen bestehenden Schliesskopf bilden. Die Anzahl der Verankerungszungen entspricht dabei der Anzahl der Kanten des polygonalen Querschnittes des Schliesselementes.

Weitere vorteilhafte Ausgestaltungen der Erfindung sind in den abhängigen Ansprüchen gekennzeichnet.

Eine andere Ausführungsform der erfindungsgemässen Vorrichtung zeichnet sich gegenüber der vorangehend beschriebenen Ausführungsform darin aus, dass das Schliesselement mindestens auf einem an das hintere Ende anschliessenden Teil seiner Länge einen quadratischen Querschnitt aufweist. Der Vorteil eines Querschnittes mit einer kleinen Kantenzahl liegt in der darin begründeten Ausbildung schärferer Kanten, wodurch die Aufteilung des Nietenschaftes in separate Verankerungszungen beim Schliessen der Blindniete begünstigt wird.

Eine andere Ausführungsform der erfindungsgemässen Vorrichtung zeichnet sich gegenüber den vorangehend beschriebenen Ausführungsformen darin aus, dass der Querschnitt des Schliesselementes mindestens auf einem an das hintere Ende anschliessenden Teil seiner Länge entlang der Längsachse gegen das hintere Ende des Schliesselementes konisch erweitert ist, wobei der Konuswinkel so gewählt ist, dass sowohl die Aufteilung des Nietenschaftes in separate Verankerungszungen als auch die Ausweitung dieser Verankerungszungen beim Schliessen der Blindniete begünstigt wird.

Eine andere Ausführungsform der erfindungsgemässen Vorrichtung zeichnet sich gegenüber den vorangehend beschriebenen Ausführungsformen darin aus, dass die Durchgangsöffnung in der Blindniete am schliessenden Teil einen quadratischen Querschnitt aufweist und an den Ecken Sollbruchstellen im Nietenschaft vorgesehen sind, wodurch die Aufteilung des Nietenschaftes in separate Verankerungszungen beim Schliessen der Blindniete begünstigt wird. Vorteilhafterweise weisen die Sollbruchstellen eine Wandstärke zwischen 1 % und 10 %, vorzugsweise zwischen 5 % und 9 % des Aussendurchmessers D der Blindniete auf.

Eine weitere Ausführungsform der erfindungsgemässen Vorrichtung unterscheidet sich von den vorangehend beschriebenen Ausführungsformen nur dadurch, dass das Schliesselement lösbar mit einem Schliessbolzen verbunden ist.

Wiederum eine weitere Ausführungsform der erfindungsgemässen Vorrichtung unterscheidet sich von den vorangehend beschriebenen Ausführungsformen dadurch, dass mindestens ein Schliesselement Teil eines Marknagels ist.

Wiederum eine weitere Ausführungsform der erfindungsgemässen Vorrichtung unterscheidet sich von den vorangehend beschriebenen Ausführungsformen dadurch, dass die Blindniete einen lösbaren Nietenkopf besitzt. Dazu kann der lösbare Nietenkopf mit einem Innengewinde ausgestattet sein, welches auf ein entsprechendes Aussengewinde auf der Blindniete aufgeschraubt werden kann. Wird die Blindniete als Anker eingesetzt, beispielsweise als Fixationsmittel im Femurkopf zusammen mit einer Seitenplatte oder einem Marknagel oder als Fadenanker, eignet sich eine Ausführung der Blindniete ohne Nietenkopf. Dabei hat die Ausführung der Blindniete ohne Nietenkopf den Vorteil, dass sie beim Spreizvorgang tiefer in den Knochen eindringen kann. Dieses "Nachrutschen" verhindert das Durchschneiden der Spongiosa beim Aufspreizen der Blindniete.

Wiederum eine weitere Ausführungsform der erfindungsgemässen Vorrichtung unterscheidet sich von den vorangehend beschriebenen Ausführungsformen dadurch, dass am hinteren Ende des Schliesselementes eine Bohrerspitze angebracht ist, wodurch ein Vorbohren des Knochens erübrigt wird.

Wiederum eine weitere Ausführungsform der erfindungsgemässen Vorrichtung unterscheidet sich von den vorangehend beschriebenen Ausführungsformen dadurch, dass die Durchgangsöffnung in der Blindniete auf einem dem zu schliessenden Teil gegenüberliegenden Teil ihrer Länge mit einem Innengewinde versehen ist, welches ein Einschrauben von beispielsweise einem anderen Implantat oder Implantatteil in die montierte Blindniete ermöglicht.

Wiederum eine weitere Ausführungsform der erfindungsgemässen Vorrichtung unterscheidet sich von den vorangehend beschriebenen Ausführungsformen dadurch, dass der Schliessbolzen des Schliesselementes elastisch biegbar ist. Dadurch wird ein Anbringen der Blindniete beispielsweise im Femurkopf ermöglicht, wobei der Schliessbolzen durch den gesamten Femurknochen hindurch gezogen werden kann.

Wiederum eine weitere Ausführungsform der erfindungsgemässen Vorrichtung zeichnet sich dadurch aus, dass die Wandstärke s am zu schliessenden Teil der Blindniete zwischen 1% und 20% des Aussendurchmessers D der Blindniete beträgt. Dadurch wird ein einfaches Aufspreizen des Nietenschaftes in die separaten Verankerungselemente mittels des polygonförmigen Konus am Schliesselement ermöglicht.

Wiederum eine weitere Ausführungsform der erfindungsgemässen Vorrichtung zeichnet sich dadurch aus, dass die Verankerungszungen mittels des Schliesselementes relativ zur Längsachse halbkreisförmig aufweitbar sind und den Schliesskopf der Blindniete bilden. Damit ergibt sich eine breite Abstützung beispielsweise auf der Knocheninnenfläche und wegen der durch die halbkreisförmige Ausbildung der Verankerungszungen nahezu senkrecht auf der Knochenfläche stehenden Enden der Verankerungszungen eine krallenartige Verankerung des Schliesskopfes.

Wiederum eine weitere Ausführungsform der erfindungsgemässen Vorrichtung zeichnet sich gegenüber der vorangehend beschriebenen Ausführungsform dadurch aus, dass die Blindniete aus einem plastisch biegbaren Material, beispielsweise aus Rein-Titan, einer Titan-Legierung oder aus Implantate-Stahl besteht, wodurch die Ausbildung der oben erwähnten halbkreisförmigen Verankerungszungen gefördert wird.

Wiederum eine weitere Ausführungsform der erfindungsgemässen Vorrichtung zeichnet sich gegenüber den vorangehend beschriebenen Ausführungsformen dadurch aus, dass zwischen zwei gegenüberliegenden Enden zweier einander gegenüberliegender Verankerungszungen des Schliesskopfes eine Länge L eingeschlossen wird, die dem 2-fachen bis 3-fachen des Durchmessers D der Blindniete entspricht. Diese grosse Aufweitung der Verankerungszungen ergibt wiederum eine breite Abstützung auf der Knocheninnenfläche.

Die durch die Erfindung erreichten Vorteile sind im wesentlichen darin zu sehen, dass dank der erfindungsgemässen Vorrichtung nur eine kleine Bohrung in den Knochen gebohrt werden muss und die erfindungsgemässe Blindniete nach der Montage eine breite Abstützung auf der Knocheninnenfläche hat. Zudem wird beispielsweise anders als bei einer Hüftschraube durch die halbkreisförmige ankerförmige Ausbildung der Verankerungszungen und deren Verankerung im Knochen die Rotation des Kopfes des Fixationsmittels verhindert. Ausserdem ist beim porotischen Knochen die Spongiosa in Gelenksköpfen, wenn überhaupt noch vorhanden, mechanisch nicht belastbar. Mit anderen Worten liegt die Hüftschraube in einem Hohlraum, was eine Dislokation des Hüftkopfes, relativ zur Hüftschraube zur Folge hat. Erst wenn die Hüftschraube Kontakt mit der Knocheninnenfläche hat, kann die Hüftschraube ihre Funktion aufnehmen. Da bei diesen Fällen das Interface zwischen Schraube und Knochen oft unzureichend ist, kommt es zu Penetrationen der Schraube in das Hüftgelenk. Bei der erfindungsgemässen Blindniete mit den halbkreisförmigen Verankerungszungen wird primär die Kopfinnenfläche als Interface zwischen Implantat und Knochen verwendet. Durch die der Anatomie gut angepasste Form der Verankerungszungen ist die Kontaktfläche zum Knochen grösser und optimaler angepasst. Wird die erfindungsgemässe Blindniete zur Verankerung eines Marknagels im Femurkopf verwendet, misst die Bohrung zur Aufnahme der erfindungsgemässen Blindniete nur ca. 8 mm. Dies hat den Vorteil, dass der Durchmesser des Marknagels, im Vergleich zu bestehenden Marknägel-Hüftschrauben Systemen, stark reduziert werden kann.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt durch eine Ausführungsform der erfindungsgemässen Blindniete und eine Ausführungsform des erfindungsgemässen Schliesselementes;
Fig. 2 einen Längsschnitt durch eine Ausführungsform der erfindungsgemässen Blindniete und eine andere Ausführungsform des erfindungsgemässen Schliesselementes;
Fig. 3 einen Querschnitt durch die in Fig. 2 gezeigte Ausführungsform des erfindungsgemässen Schliesselementes;
Fig. 4 einen Längsschnitt durch eine Ausführungsform einer geschlossenen erfindungsgemässen Blindniete mit einer Knochenplatte und einem Knochenstück;
Fig. 5 eine Ansicht der in Fig. 4 dargestellten Ausführungsform einer geschlossenen erfindungsgemässen Blindniete;
Fig. 6 einen Längsschnitt durch eine weitere Ausführungsform der erfindungsgemässen Blindniete;
Fig. 7 eine Frontansicht der in Fig. 6 dargestellten Ausführungsform der erfindungsgemässen Blindniete;
Fig. 8 einen Längsschnitt durch eine weitere Ausführungsform der erfindungsgemässen Blindniete;
Fig. 9 einen Längsschnitt durch eine weitere Ausführungsform der erfindungsgemässen Blindniete und eine weitere Ausführungsform des erfindungsgemässen Schliesselementes;
Fig. 10 eine Frontansicht der in Fig. 9 dargestellten Ausführungsform der erfindungsgemässen Blindniete mit dem in Fig. 9 dargestellten erfindungsgemässen Schliesselement; und
Fig. 11 einen Längsschnitt durch einen Femurknochen mit einer erfindungsgemässen Blindniete, einem Marknagel und einer Montagehülse.

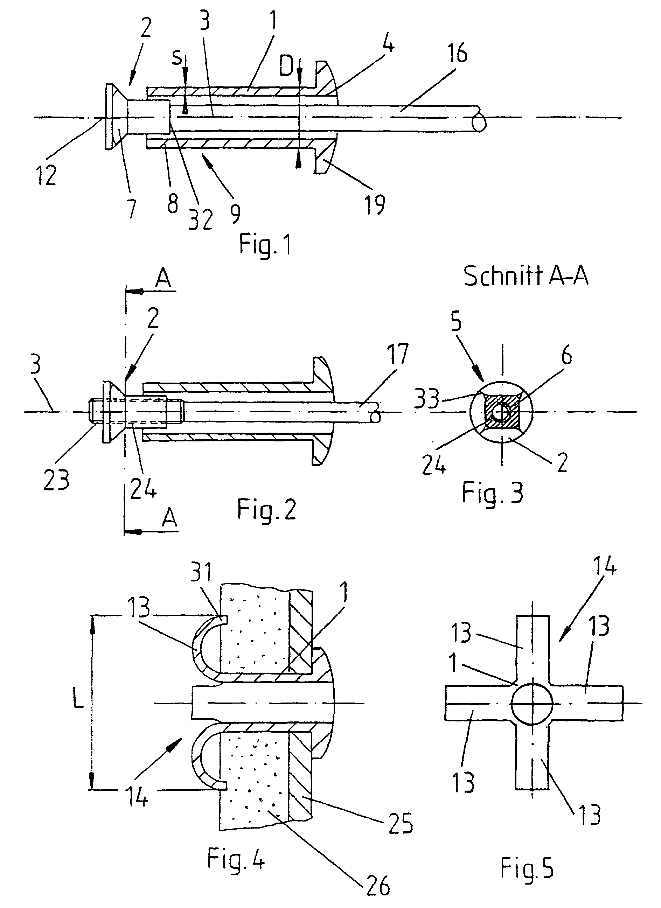

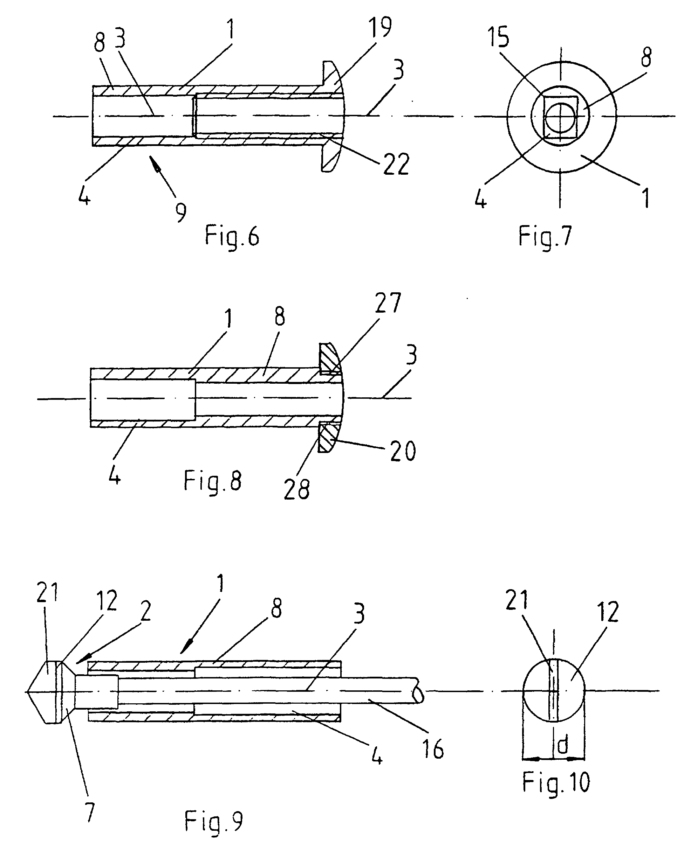

In Fig. 1 sind eine Blindniete 1 und ein Schliesselement 2 gemäss einer Ausführungsform der erfindungsgemässen Vorrichtung dargestellt. Die Blindniete 1 weist eine Längsachse 3 auf und besteht aus einem parallel zur Längsachse 3 verlaufenden zylindrischen Nietenschaft 8, der nicht zwangsläufig kreiszylindrisch sein muss, einem fest mit dem Nietenschaft 8 verbundenen Nietenkopf 19 und einer entlang der Längsachse 3 koaxial die Blindniete 1 durchdringenden zylindrischen Durchgangsöffnung 4. Der Nietenschaft 8 weist einen Aussendurchmesser D auf, so dass die durch die Weite der Durchgangsöffnung 4 und dem Aussendurchmesser D definierte Wandstärke so bemessen ist, dass beim Schliessen der Blindniete 1 mit dem Schliesselement 2 die Blindniete 1 auf dem zu schliessenden Teil 9 in separate Verankerungszungen 13 (Fig. 4 und 5) trennbar ist, wobei die Anzahl der Verankerungszungen 13 der Zahl der Kanten des polygonalen Querschnittes 5 des Schliesselementes 2 (Fig. 3) entspricht. Bei der hier dargestellten Ausführungsform der Blindniete 1 entspricht die Wandstärke s des Nietenschaftes 8 14% des Aussendurchmessers D. Bei der in Fig. 1 dargestellten Ausführungsform der erfindungsgemässen Vorrichtung ist das Schliesselement 2 Bestandteil eines Schliessbolzens 16. Das Schliesselement 2 weist auf seinem an sein hinteres Ende 12 anschliessenden Teil seiner Länge einen polygonalen, gegen das hintere Ende 12 weiter werdenden Querschnitt 5 (Fig. 3) auf. Das Schliessen der Blindniete 1 erfolgt nach dem Einführen mittels des Schliesselementes 2, welches mittels einer auf den Schliessbolzen 16 ausgeübten Zugkraft in den zu schliessenden Teil 9 der Blindniete 1 eingepresst wird. Beim Einpressen des weiter werdenden polygonförmigen Teils 7 des Schliesselementes 2 wird die Wand des Nietenschaftes 8 aufgeweitet und durch die Kanten des polygonartigen Querschnitts 5 (Fig. 3) in die Verankerungszungen 13 (Fig. 4 und 5) aufgetrennt. Der Schliessbolzen 16 kann mittels einer Sollbruchstelle mit dem Schliesselement 2 verbunden werden, wodurch der Schliessbolzen 16 nach dem Schliessen der Blindniete 1 beispielsweise durch Aufbringen von Torsionskräften vom Schliesselement 2 abgetrennt werden kann.

Die in Fig. 2 dargestellte Ausführungsform der erfindungsgemässen Vorrichtung unterscheidet sich von der in Fig. 1 dargestellten Ausführungsform nur darin, dass das Schliesselement 2 ein koaxial zur Längsachse 3 verlaufendes Innengewinde 24 aufweist und der Schliessbolzen 17 ein entsprechendes Aussengewinde 23 aufweist, wodurch der als separates Element gestaltete Schliessbolzen 17 lösbar in das Schliesselement 2 einschraubbar ist. Nach dem Schliessen der Blindniete 1 kann der Schliessbolzen 17 aus dem Schliesselement 2 herausgeschraubt werden und somit von der geschlossenen Blindniete 1 entfernt werden.

Fig. 3 zeigt einen Schnitt senkrecht zur Längsachse 3 durch das Schliesselement 2. In der hier gezeigten Ausführungsform der erfindungsgemässen Vorrichtung besteht der polygonförmige Querschnitt 5 aus einem quadratischen Querschnitt 6, wodurch sich beim Schliessen der Blindniete 1 vier Verankerungszungen 13 ausbilden. Ebenfalls in Fig. 3 gezeigt ist das Innengewinde 24 des Schliesselementes 2 gemäss der in Fig. 2 dargestellten Ausführungsform der erfindungsgemässen Vorrichtung.

Fig. 4 zeigt einen Längsschnitt durch eine geschlossene Blindniete 1 gemäss einer Ausführungsform der erfindungsgemässen Vorrichtung. Die Blindniete 1 verbindet eine Knochenplatte 25 mit einem Knochen 26. Der Schliesskopf 14 umfasst vier Verankerungszungen 13, deren Enden 31 die Länge L einschliessen. Aus dieser Darstellung ist auch ersichtlich, dass die von den Enden 31 der Verankerungszungen 13 eingeschlossene Länge L etwa dem 3-fachen des Durchmessers D des Nietenschaftes 8 entspricht. Eine Ansicht der in Fig. 4 dargestellten geschlossenen Blindniete 1 mit vier Verankerungszungen 13 ist in Fig. 5 gezeigt.

Die in den Fig. 6 und 7 gezeigte Blindniete 1 gemäss einer weiteren Ausführungsform der erfindungsgemässen Vorrichtung unterscheidet sich von der in Fig. 1 dargestellten Ausführungsform nur darin, dass die Durchgangsöffnung 4 auf dem vom Nietenkopf 19 entfernten, schliessenden Teil 9 der Blindniete 1 quadratisch ausgeführt ist. Die Ecken 15 der Durchgangsöffnung 4 bilden zwischen Durchgangsöffnung 4 und äusserem Durchmesser D des Nietenschaftes 8 Sollbruchstellen, an denen der Nietenschaft 8 beim Schliessen der Blindniete 1 in die Verankerungszungen 13 (Fig. 4 und 5) aufgetrennt wird. Am an den Nietenkopf 19 anschliessenden Teil der Blindniete 1 ist die Durchgangsöffnung 4 entsprechend der hier dargestellten Ausführungsform der erfindungsgemässen Vorrichtung koaxial zur Längsachse 3 mit einem Innengewinde 22 versehen.

Die in Fig. 8 dargestellte Blindniete 1 gemäss einer weiteren Ausführungsform der erfindungsgemässen Vorrichtung unterscheidet sich von den in den Fig. 1 - 7 gezeigten Ausführungsformen nur darin, dass der Nietenkopf 20 lösbar mit dem Nietenschaft 8 der Blindniete 1 verbunden ist. Diese lösbare Verbindung zwischen Nietenkopf 20 und Nietenschaft 8 ist durch eine Gewindeverbindung ausgeführt. Dazu ist der Nietenschaft 8 mit einem koaxial zur Längsachse 3 verlaufenden Aussengewinde 27 und der Nietenkopf 20 mit einem entsprechenden Innengewinde 28 versehen.

Die Fig. 9 und 10 zeigen eine weitere Ausführungsform der erfindungsgemässen Vorrichtung. Die ebenfalls zylindrisch ausgebildete Blindniete 1 hat keinen Nietenkopf und besteht aus einem zylindrischen Nietenschaft 8 mit der Längsachse 3 und einer zur Längsachse 3 koaxialen ebenfalls zylindrischen Durchgangsöffnung 4. Das Schliesselement 2 unterscheidet sich vor der in Fig. 1 dargestellten Schliesselement 2 nur darin, dass das hintere Ende 12 des Schliesselementes 2 mit einem Bohrelement 21 versehen ist. Der Durchmesser d des Bohrelements 21 entspricht dem Aussendurchmesser D der Blindniete 1, so dass bei der Montage der Blindniete 1 der Knochen 26 (Fig. 4) nicht vorgebohrt werden muss. Anstelle des in Fig. 9 und 10 gezeigten Spitzbohrers 21 ist selbstverständlich auch ein Spiralbohrer möglich.

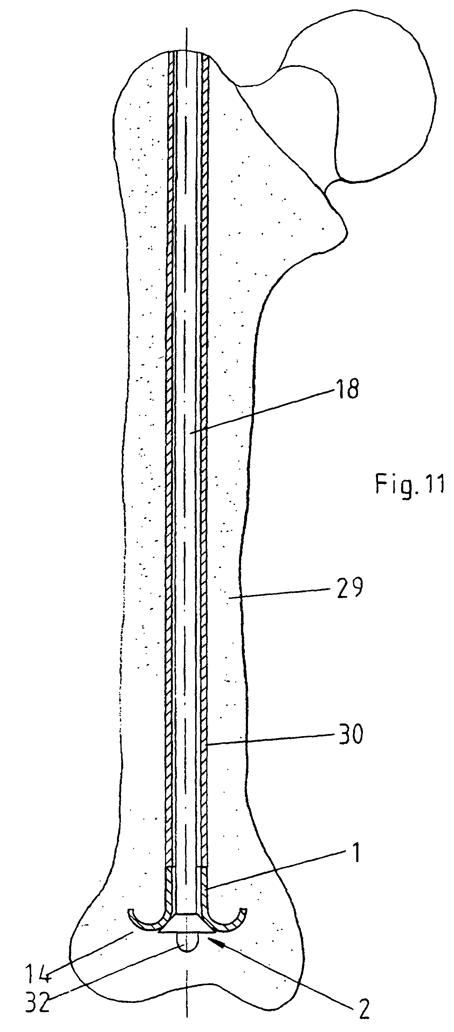

Fig. 11 zeigt eine Anwendung der Blindniete 1 gemäss einer Ausführungsform der erfindungsgemässen Vorrichtung zur Verriegelung eines Marknagels 18 in beispielsweise einem Femurknochen 29. Durch Einbringen der Blindniete 1 wird der Marknagel 18 axial in proximaler Richtung gesichert. Das Schliesselement 2 ist am distalen Ende 32 des Marknagels 18 als Bestandteil des Marknagels 18 angebracht. Zur Montage der Blindniete 1 wird diese zwischen dem Schliesselement 2 und einer Hülse 30 eingeklemmt und in den Femurknochen 29 zusammen mit dem Marknagel 18 eingeführt. Durch Aufbringen einer Zugkraft auf den Marknagel 18 in proximaler Richtung und einer entgegengesetzten Haltekraft auf die Hülse 30 wird der Schliesskopf 14 an der Blindniete 1 geformt und die Blindniete 1 verriegelt.

## Patentansprüche

1. Vorrichtung zur Fixation von chirurgischen Implantaten, chirurgischen Fäden oder Geweben im oder am Knochen, wobei
A) die Vorrichtung eine Blindniete (1) mit einer Längsachse (3) und einer koaxialen Durchgangsöffnung (4) sowie ein Schliesselement (2) mit einem hinteren Ende (12) und einem vorderen Ende (32) umfasst; wobei
B) beim Schliesselement (2) mindestens auf einem an das hintere Ende (12) anschliessenden Teil (7) seiner Länge der Querschnitt (5) entlang der Längsachse (3) gegen das hintere Ende (12) weiter wird;
**dadurch gekennzeichnet, dass**
C) das Schliesselement (2) mindestens auf einem an das hintere Ende (12) anschliessenden Teil (7) seiner Länge einen polygonalen Querschnitt (5) aufweist;
D) beim Schliessen der Blindniete (1) diese durch Einpressen des Schliesselementes (2) auf dem zu schliessenden Teil (9) der Blindniete (1) in separate Verankerungszungen (13) trennbar ist;
E) die Verankerungszungen (13) mittels des Schliesselementes (2) relativ zur Längsachse (3) aufweitbar sind und den Schliesskopf (14) der Blindniete (1) bilden;
F) die Durchgangsöffnung (4) in der Blindniete (1) am schliessenden Teil (9) einen polygonalen Querschnitt aufweist; und
G) an den Ecken (15) des polygonalen Querschnitts Sollbruchstellen im oder auf dem Nietenschaft (8) vorgesehen sind.

2. Vorrichtung nach Anspruch 1, **dadurch gekennzeichnet, dass** das Schliesselement (2) mindestens auf einem an das hintere Ende (12) anschliessenden Teil (7) seiner Länge einen polygonalen Querschnitt (5) aufweist.

3. Vorrichtung nach Anspruch 1 oder 2, **dadurch gekennzeichnet, dass** der Querschnitt (5) des Schliesselementes (2) mindestens auf einem an das hintere Ende (12) anschliessenden Teil seiner Länge entlang der Längsachse (3) gegen das hintere Ende (12) des Schliesselementes (2) konisch erweitert ist.

4. Vorrichtung nach einem der Ansprüche 1 bis 3, **dadurch gekennzeichnet, dass** das Schliesselement (2) Bestandteil eines Schliessbolzens (16) ist.

5. Vorrichtung nach einem der Ansprüche 1 bis 3, **dadurch gekennzeichnet, dass** das Schliesselement (2) lösbar mit einem Schliessbolzen (17) verbunden ist.

6. Vorrichtung nach einem der Ansprüche 1 bis 3, **dadurch gekennzeichnet, dass** mindestens ein Schliesselement (2) Teil eines Marknagels (18) ist.

7. Vorrichtung nach einem der Ansprüche 1 bis 6, **dadurch gekennzeichnet, dass** die Blindniete (1) einen festen Nietenkopf (19) besitzt.

8. Vorrichtung nach einem der Ansprüche 1 bis 6, **dadurch gekennzeichnet, dass** die Blindniete (1) einen lösbaren Nietenkopf (20) besitzt.

9. Vorrichtung nach Anspruch 8, **dadurch gekennzeichnet, dass** der lösbare Nietenkopf (20) ein Innengewinde (28) aufweist und die Blindniete (1) ein entsprechendes Aussengewinde (27) aufweist.

10. Vorrichtung nach einem der Ansprüche 1 bis 9, **dadurch gekennzeichnet, dass** am hinteren Ende (12) des Schliesselementes (2) eine Bohrerspitze (21) angebracht ist.

11. Vorrichtung nach einem der Ansprüche 1 bis 10, **dadurch gekennzeichnet, dass** die Durchgangsöffnung (4) in der Blindniete (1) auf einem dem zu schliessenden Teil (9) gegenüberliegenden Teil ihrer Länge mit einem Innengewinde (22) versehen ist.

12. Vorrichtung nach einem der Ansprüche 1 bis 11, **dadurch gekennzeichnet, dass** der Schliessbolzen (16;17) des Schliesselementes (2) deformierbar biegbar ist.

13. Vorrichtung nach einem der Ansprüche 1 bis 11, **dadurch gekennzeichnet, dass** der Schliessbolzen (16;17) des Schliesselementes (2) elastisch biegbar ist.

14. Vorrichtung nach einem der Ansprüche 1 bis 13, **dadurch gekennzeichnet, dass** die Wandstärke s am zu schliessenden Teil (9) der Blindniete (1) zwischen 1 % und 20 % des Aussendurchmessers D der Blindniete (1) beträgt.

15. Vorrichtung nach einem der Ansprüche 1 bis 14, **dadurch gekennzeichnet, dass** die Verankerungszungen (13) mittels des Schliesselementes (2) relativ zur Längsachse (3) halbkreisförmig aufweitbar sind und den Schliesskopf (14) der Blindniete (1) bilden.

16. Vorrichtung nach einem der Ansprüche 1 bis 15, **dadurch gekennzeichnet, dass** die Sollbruchstellen (15) eine Wandstärke zwischen 1 % und 10 % des Aussendurchmessers D der Blindniete (1) aufweisen.

17. Vorrichtung nach einem der Ansprüche 1 bis 15, **dadurch gekennzeichnet, dass** die Sollbruchstellen (15) eine Wandstärke zwischen 5 % und 9 % des Aussendurchmessers D der Blindniete (1) aufweisen.

18. Vorrichtung nach einem der Ansprüche 1 bis 17, **dadurch gekennzeichnet, dass** zwischen zwei gegenüberliegenden Enden (31) des Schliesskopfes (14) eine Länge L eingeschlossen wird, die dem 2-fachen bis 3-fachen des Durchmessers D der Blindniete (1) entspricht.

19. Vorrichtung nach einem der Ansprüche 1 bis 17, **dadurch gekennzeichnet, dass** die Wandstärke s am zu schliessenden Teil (9) der Blindniete (1) sich in Richtung gegen den Nietenkopf (19;20) hin verdickt.

## Claims

1. A fastener to affix surgical implants, sutures or tissues in or at the bone, where
A) the fastener comprises a blind rivet (1) having a longitudinal axis (3) and a coaxial passage (4), further a closing element (2) having a rear end (12) and a front end (32); whereby
B) the cross-section (5) of the closing element (2) flares along the longitudinal axis (3) toward the rear end (12) at least on a segment (7) adjoining its rear end (12),
**characterized in that**
C) the closing element (2) is of polygonal cross-section (5) at least on a segment (7) adjoining its rear end (12),
D) the blind rivet (1) when being closed being severable into separate anchoring legs (13) by the closing element (2) being pressed into the part (9) to be closed of the blind rivet (1),
E) the anchoring legs (13) can be spread by the closing element (2) relative to the longitudinal axis (3) and form the closing head (14) of the blind rivet (1);
F) the cross-section of the passage (4) at the closing part (9) of the blind rivet (1) is polygonal,
G) design rupture sites are present in or on the rivet shank (8) at the corners (15) of the polygonal cross-section.

2. Fastener as claimed in claim 1, **characterized in that** the closing element (2) is of polygonal cross-section (6) at least on a segment (7) of its length adjoining the rear end (12).

3. Fastener as claimed in claim 1 or 2, **characterized in that** the cross-section (5) of the closing element (2) flares conically along the longitudinal axis (3) toward the rear end (12) of the closing element (2) at least on one segment of its length adjoining the rear end (12).

4. Fastener as claimed in one of claims 1 through 3, **characterized in that** the closing element (2) is a component of a closing pin (16).

5. Fastener as claimed in one of claims 1 through 3, **characterized in that** the closing element (2) is detachably connected to the closing pin (17).

6. Fastener as claimed in one of claims 1 through 3, **characterized in that** at least one closing element (2) is part of a marrow spindle (18).

7. Fastener as claimed in one of claims 1 through 6, **characterized in that** the blind rivet (1) comprises an integral rivet head (19)

8. Fastener as claimed in one of claims 1 through 6, **characterized in that** the blind rivet (1) comprises a detachable rivet head (20).

9. Fastener as claimed in claim 8, **characterized in that** the detachable rivet head (20) comprises an inside thread (28) and **in that** the blind rivet (1) comprises a matching outside thread (27).

10. Fastener as claimed in one of claims 1 through 9, **characterized in that** an awl (21) is mounted at the rear end (12) of the closing element (2).

11. Fastener as claimed in one of claims 1 through 10, **characterized in that** the passage (4) in the blind rivet (1) is fitted with an inside thread (22) on a segment of its length opposite the part (9) of the rivet to be closed.

12. Fastener as claimed in one of claims 1 through 11, **characterized in that** the closing pin (16; 17) of the closing element (2) can be bent in deforming manner.

13. Fastener as claimed in one of claims 1 through 11, **characterized in that** the closing pin (16; 17) of the closing element (2) is elastically bending.

14. Fastener as claimed in one of claims 1 through 13, **characterized in that** the wall thickness "s" at the closing part (9) of the blind rivet (1) is between 1 and 20 % of the outside diameter D of the blind rivet (1).

15. Fastener as claimed in one of claims 1 through 14, **characterized in that** the anchoring legs (13) can be widened in semi-circular manner relative to the longitudinal axis (3) by the closing element (2) and **in that** they form the closing head (14) of the blind rivet (1).

16. Fastener as claimed in one of claims 1 through 15, **characterized in that** the design rupture sites (15) are of a wall thickness between 1 and 10 % the diameter D of the blind rivet (1).

17. Fastener as claimed in one of claims 1 through 15, **characterized in that** the design rupture sites (15) are of a wall thickness between 5 and 9 % the diameter D of the blind rivet (1).

18. Fastener as claimed in one of claims 1 through 17, **characterized in that** two mutually opposite ends (31) of the closing head (14) subtend a distance L twice to three times the diameter D of the blind rivet (1).

19. Fastener as claimed in one of claims 1 through 17, **characterized in that** the wall thickness "s" becomes thicker in the direction of the rivet head (19; 20) at the part (9) of the blind rivet (1) to be closed.

## Revendications

1. Dispositif permettant de fixer des implants chirurgicaux, des fils chirurgicaux ou des tissus dans ou sur l'os,
A) le dispositif comprenant un rivet aveugle (1) avec un axe longitudinal (3) et un trou de passage coaxial (4) ainsi qu'un élément de fermeture (2) avec une extrémité arrière (12) et une extrémité avant (32);
B) l'élément de fermeture (2) présentant, au moins sur la partie (7) de sa longueur située dans le prolongement de l'extrémité arrière (12), une section (5) s'élargissant le long de l'axe longitudinal (3) vers l'extrémité arrière (12);
**caractérisé en ce que**
C) l'élément de fermeture (2) présente, au moins sur une partie (7) de sa longueur située dans le prolongement de l'extrémité arrière (12), une section polygonale (5);
D) lors de la fermeture du rivet aveugle (1), celui-ci peut être divisé en languettes d'ancrage séparées (13), par enfoncement de l'élément de fermeture (2) sur la partie (9) du rivet aveugle (1) devant assurer la fermeture;
E) les languettes d'ancrage (13) peuvent être élargies par rapport à l'axe longitudinal (3) au moyen de l'élément de fermeture (2) de manière à former la tête de fermeture (14) du rivet aveugle (1);
F) le trou de passage (4) ménagé dans le rivet aveugle (1) présente une section polygonale sur la partie (9) assurant la fermeture; et
G) des points destinés à la rupture sont prévus, au niveau des angles (15) de la section polygonale, dans ou sur le corps de rivet (8).

2. Dispositif selon la revendication 1, **caractérisé en ce que** l'élément de fermeture (2) présente, au moins sur une partie (7) de sa longueur située dans le prolongement de l'extrémité arrière (12), une section polygonale (5);

3. Dispositif selon la revendication 1 ou 2, **caractérisé en ce que** la section (5) de l'élément de fermeture (2) est, au moins sur une partie de sa longueur située dans le prolongement de l'extrémité arrière (12), élargie de manière conique, le long de l'axe longitudinal (3), vers l'extrémité arrière (12) de l'élément de fermeture (2).

4. Dispositif selon l'une des revendications 1 à 3, **caractérisé en ce que** l'élément de fermeture (2) fait partie d'un boulon de fermeture (16).

5. Dispositif selon l'une des revendications 1 à 3, **caractérisé en ce que** l'élément de fermeture (2) est relié de manière amovible à un boulon de fermeture (17).

6. Dispositif selon l'une des revendications 1 à 3, **caractérisé en ce qu'**au moins un élément de fermeture (2) fait partie d'un clou intramédullaire (18).

7. Dispositif selon l'une des revendications 1 à 6, **caractérisé en ce que** le rivet aveugle (1) possède une tête de rivet fixe (19).

8. Dispositif selon l'une des revendications 1 à 6, **caractérisé en ce que** le rivet aveugle (1) possède une tête de rivet amovible (20).

9. Dispositif selon la revendication 8, **caractérisé en ce que** la tête de rivet amovible (20) présente un filet intérieur (28) et **en ce que** le rivet aveugle (1) présente un filet extérieur (27) correspondant.

10. Dispositif selon l'une des revendications 1 à 9, **caractérisé en ce qu'**un foret (21) est monté sur l'extrémité arrière (12) de l'élément de fermeture (2).

11. Dispositif selon l'une des revendications 1 à 10, **caractérisé en ce que** le trou de passage (4) ménagé dans le rivet aveugle (1) est pourvu, sur une partie de sa longueur située à l'opposé de la partie (9) devant assurer la fermeture, d'un filet intérieur (22).

12. Dispositif selon l'une des revendications 1 à 11, **caractérisé en ce que** le boulon de fermeture (16,17) de l'élément de fermeture est flexible de manière à pouvoir subir une déformation plastique.

13. Dispositif selon l'une des revendications 1 à 11, **caractérisé en ce que** le boulon de fermeture (16,17) de l'élément de fermeture (2) est flexible de manière à pouvoir subir une déformation élastique.

14. Dispositif selon l'une des revendications 1 à 13, **caractérisé en ce que** l'épaisseur de paroi s est comprise, au niveau de la partie (9) du rivet aveugle (1) devant assurer la fermeture, entre 1 % et 20 % du diamètre extérieur D du rivet aveugle (1).

15. Dispositif selon l'une des revendications 1 à 14, **caractérisé en ce que** les languettes d'ancrage (13) peuvent être élargies en demi-cercle par rapport à l'axe longitudinal (3) au moyen de l'élément de fermeture (2) de manière à former la tête de fermeture (14) du rivet aveugle (1).

16. Dispositif selon l'une des revendications 1 à 15, **caractérisé en ce que** les points (15) destinés à la rupture présentent une épaisseur de paroi comprise entre 1 % et 10 % du diamètre extérieur D du rivet aveugle (1).

17. Dispositif selon l'une des revendications 1 à 15, **caractérisé en ce que** les points (15) destinés à la rupture présentent une épaisseur de paroi comprise entre 5 % et 9 % du diamètre extérieur D du rivet aveugle (1).

18. Dispositif selon l'une des revendications 1 à 17, **caractérisé en ce que**, entre deux extrémités opposées (31) de la tête de fermeture (14), est comprise une longueur L pouvant aller du double au triple du diamètre D du rivet aveugle (1).

19. Dispositif selon l'une des revendications 1 à 17, **caractérisé en ce que**, au niveau de la partie (9) du rivet aveugle (1) devant assurer la fermeture, l'épaisseur de paroi s va en augmentant en direction de la tête de rivet (19;20).
